(19) Europäisches Patentamt — European Patent Office — Office européen des brevets

(11) **EP 2 649 090 B1**

(12) **FASCICULE DE BREVET EUROPEEN**

(45) Date de publication et mention
de la délivrance du brevet:
**22.08.2018 Bulletin 2018/34**

(21) Numéro de dépôt: **11805206.7**

(22) Date de dépôt: **05.12.2011**

(51) Int Cl.:
*C07K 7/06* (2006.01)     *C07K 7/08* (2006.01)
*C07K 14/47* (2006.01)     *C12N 9/64* (2006.01)

(86) Numéro de dépôt international:
**PCT/IB2011/055461**

(87) Numéro de publication internationale:
**WO 2012/077037 (14.06.2012 Gazette 2012/24)**

(54) **PEPTIDES MODULATEURS DU COMPLEXE SASPASE-FLG2**

PEPTIDE ALS MODULATOREN DES SASPASE-FLG2-KOMPLEXES

PEPTIDES THAT MODULATE COMPLEX SASPase-FLG2

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **07.12.2010 FR 1060177
23.12.2010 US 201061457090 P**

(43) Date de publication de la demande:
**16.10.2013 Bulletin 2013/42**

(73) Titulaire: **L'Oréal
75008 Paris (FR)**

(72) Inventeurs:
• **BERNARD, Dominique
F-92170 Vanves (FR)**
• **THOMAS-COLLIGNON, Agnès
F-78180 Montigny Le Bretonneux (FR)**

(74) Mandataire: **Nony
11 rue Saint-Georges
75009 Paris (FR)**

(56) Documents cités:
**EP-A1- 1 935 243     FR-A1- 2 842 209**

• **TOULZA EVE ET AL: "Large-scale identification
of human genes implicated in epidermal barrier
function", GENOME BIOLOGY, BIOMED
CENTRAL LTD., LONDON, GB, vol. 8, no. 6, 11
juin 2007 (2007-06-11), page R107, XP021031239,
ISSN: 1465-6906, DOI:
DOI:10.1186/GB-2007-8-6-R107**
• **WU ZHIHONG ET AL: "Molecular Identification
and Expression Analysis of Filaggrin-2, a Member
of the S100 Fused-Type Protein Family", PLOS
ONE, vol. 4, no. 4, avril 2009 (2009-04),
XP009150055, ISSN: 1932-6203**

SEQUENCE LISTING

<110> L'OREAL

<120> Peptides modulateurs du complexe SASPase-FLG2

<130> BR94527/HG/ca

<150> FR1060177
<151> 2010-12-07

<150> US61457090
<151> 2010-12-23

<160> 16

<170> BiSSAP 1.0

<210> 1
<211> 285
<212> DNA
<213> Homo sapiens

<220>
<221> source
<222> 1..285
<223> /mol_type="DNA" /organism="Homo sapiens"

<400> 1

```
accgacctct tgagaagtgt tgtcaccgta attgatgttt tctacaaata caccaagcaa        60

gatggggagt gtggcacact gagcaagggt gaactaaagg aacttctgga gaaagagctt       120

catccagttc tgaagaaccc agatgatcca gacacagtgg atgtcatcat gcatatgctg       180

gatcgagatc atgacagaag attggacttt actgagtttc ttttgatgat attcaagctg       240

actatggcct gcaacaaggt cctcagcaaa gaatactgca aagct                       285
```

<210> 2
<211> 60
<212> DNA
<213> Homo sapiens

<220>
<221> source
<222> 1..60
<223> /mol_type="DNA" /organism="Homo sapiens"

<400> 2
tttactgagt ttcttttgat gatattcaag ctgactatgg cctgcaacaa ggtcctcagc        60

<210> 3
<211> 45
<212> DNA
<213> Homo sapiens

<220>
<221> source
<222> 1..45
<223> /mol_type="DNA" /organism="Homo sapiens"

<400> 3
gagtttcttt tgatgatatt caagctgact atggcctgca acaag        45

<210> 4
<211> 45
<212> DNA
<213> Homo sapiens

<220>
<221> source
<222> 1..45
<223> /mol_type="DNA" /organism="Homo sapiens"

<400> 4
ctcatgatat tcaagctgac tatggcctgc aacaaggtcc tcagc        45

<210> 5
<211> 39
<212> DNA
<213> Homo sapiens

<220>
<221> source
<222> 1..39
<223> /mol_type="DNA" /organism="Homo sapiens"

<400> 5
ctcatgatat tcaagctgac tatggcctgc aacaaggtc          39


<210> 6
<211> 48
<212> DNA
<213> Homo sapiens


<220>
<221> source
<222> 1..48
<223> /mol_type="DNA" /organism="Homo sapiens"


<400> 6
actgagtttc ttttgatgat attcaagctg actatggcct gcaacaag          48


<210> 7
<211> 24
<212> DNA
<213> Homo sapiens


<220>
<221> source
<222> 1..24
<223> /mol_type="DNA" /organism="Homo sapiens"


<400> 7
actgagtttc ttttgatgat attc          24


<210> 8
<211> 231
<212> DNA
<213> Homo sapiens


<220>
<221> source
<222> 1..231
<223> /mol_type="DNA" /organism="Homo sapiens"


<400> 8

cggcagcatg ccttcgtccc ggaacctttt gatggggcca atgtcgtccc aaacctctgg          60

ctgcacagct ttgaagtcat caatgacctc aaccattggg accatatcac caagctaagg          120

ttcctgaaag agtccctcag aggagaggcc ctgggtgtct acaataggct cagtccccag          180

gaccagggag actatgggac tgtgaaagag gccctcctga aggcctttgg g          231


<210> 9
<211> 95
<212> PRT
<213> Homo sapiens


<220>
<221> SOURCE
<222> 1..95
<223> /mol_type="protein" /organism="Homo sapiens"

<400> 9

```
Thr Asp Leu Leu Arg Ser Val Val Thr Val Ile Asp Val Phe Tyr Lys
1               5                   10                  15
Tyr Thr Lys Gln Asp Gly Glu Cys Gly Thr Leu Ser Lys Gly Glu Leu
            20                  25                  30
Lys Glu Leu Leu Glu Lys Glu Leu His Pro Val Leu Lys Asn Pro Asp
        35                  40                  45
Asp Pro Asp Thr Val Asp Val Ile Met His Met Leu Asp Arg Asp His
    50                  55                  60
Asp Arg Arg Leu Asp Phe Thr Glu Phe Leu Leu Met Ile Phe Lys Leu
65                  70                  75                  80
Thr Met Ala Cys Asn Lys Val Leu Ser Lys Glu Tyr Cys Lys Ala
            85                  90                  95
```

<210> 10
<211> 20
<212> PRT
<213> Homo sapiens

<220>
<221> SOURCE
<222> 1..20
<223> /mol_type="protein" /organism="Homo sapiens"

<400> 10

```
Phe Thr Glu Phe Leu Leu Met Ile Phe Lys Leu Thr Met Ala Cys Asn

    1               5                   10                  15
Lys Val Leu Ser
            20
```

<210> 11
<211> 15
<212> PRT
<213> Homo sapiens

<220>
<221> SOURCE
<222> 1..15
<223> /mol_type="protein" /organism="Homo sapiens"

<400> 11

```
Glu Phe Leu Leu Met Ile Phe Lys Leu Thr Met Ala Cys Asn Lys
1               5                   10                  15
```

<210> 12
<211> 15
<212> PRT
<213> Homo sapiens

<220>
<221> SOURCE
<222> 1..15
<223> /mol_type="protein" /organism="Homo sapiens"

<400> 12

        Leu Met Ile Phe Lys Leu Thr Met Ala Cys Asn Lys Val Leu Ser
        1               5                   10                  15

<210> 13
<211> 13
<212> PRT
<213> Homo sapiens

<220>
<221> SOURCE
<222> 1..13
<223> /mol_type="protein" /organism="Homo sapiens"

<400> 13

        Leu Met Ile Phe Lys Leu Thr Met Ala Cys Asn Lys Val
        1               5                   10

<210> 14
<211> 16
<212> PRT
<213> Homo sapiens

<220>
<221> SOURCE
<222> 1..16
<223> /mol_type="protein" /organism="Homo sapiens"

<400> 14

        Thr Glu Phe Leu Leu Met Ile Phe Lys Leu Thr Met Ala Cys Asn Lys
        1               5                   10                  15

<210> 15
<211> 8
<212> PRT
<213> Homo sapiens

<220>
<221> SOURCE
<222> 1..8
<223> /mol_type="protein" /organism="Homo sapiens"

<400> 15

        Thr Glu Phe Leu Leu Met Ile Phe
        1               5

<210> 16
<211> 77
<212> PRT
<213> Homo sapiens

<220>
<221> SOURCE
<222> 1..77

<223> /mol_type="protein" /organism="Homo sapiens"

<400> 16

```
Arg Gln His Ala Phe Val Pro Glu Pro Phe Asp Gly Ala Asn Val Val
1               5               10              15
Pro Asn Leu Trp Leu His Ser Phe Glu Val Ile Asn Asp Leu Asn His
            20              25              30
Trp Asp His Ile Thr Lys Leu Arg Phe Leu Lys Glu Ser Leu Arg Gly
        35              40              45
Glu Ala Leu Gly Val Tyr Asn Arg Leu Ser Pro Gln Asp Gln Gly Asp
    50              55              60
Tyr Gly Thr Val Lys Glu Ala Leu Leu Lys Ala Phe Gly
65              70              75
```

**Description**

La présente invention concerne une nouvelle cible cosmétique ou thérapeutique à l'égard de la peau et/ou de ses annexes, ainsi que sa mise en oeuvre à des fins de marqueur biologique de la peau ou pour le criblage de nouveaux actifs dédiés au soin cosmétique, thérapeutique ou dermatologique de la peau et/ou de ses annexes.

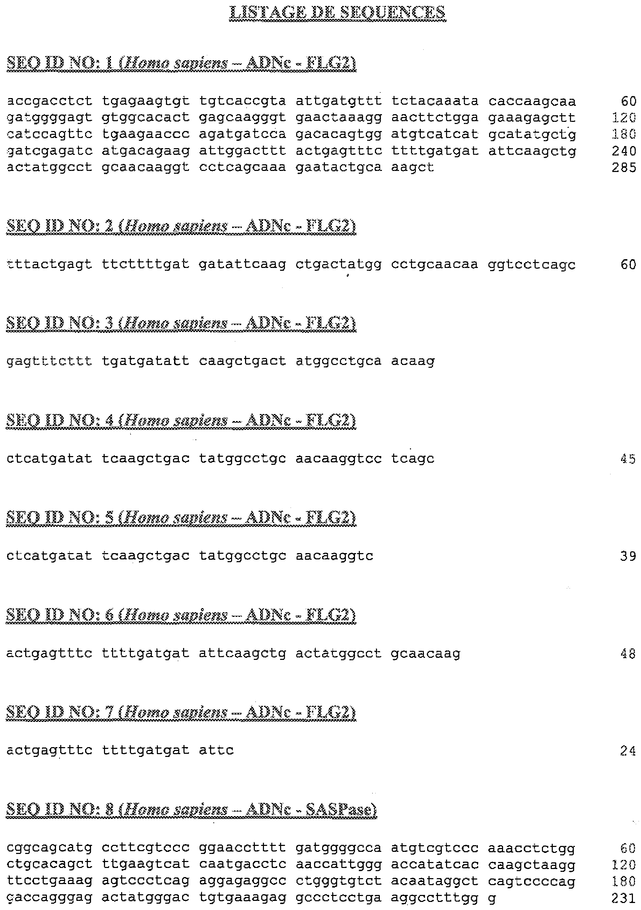

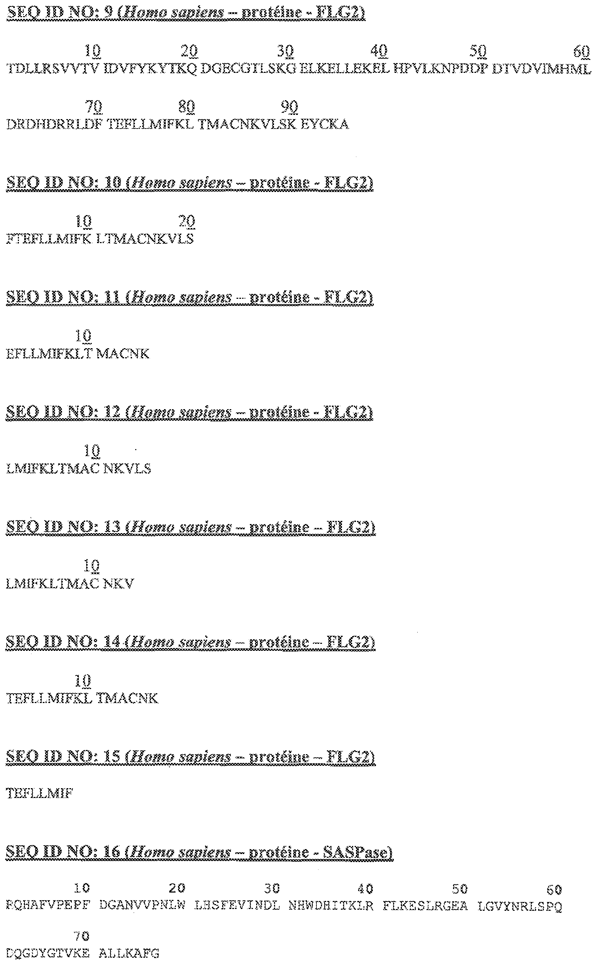

Plus précisément, la présente invention concerne des peptides isolés, issus respectivement de la Filaggrine-2, ou FLG2, et de la SASPase, aptes à former ensemble un complexe, et leur mise en oeuvre à titre de cible pour le criblage d'actifs cosmétiques ou thérapeutiques à l'égard de la peau et/ou de ses annexes.

Par « la peau et ses annexes », on entend l'ensemble de la peau du corps, le cuir chevelu, les muqueuses, et notamment les lèvres, les poils, les cheveux et les ongles. Plus particulièrement, la présente invention concerne la peau du corps, le cuir chevelu, les lèvres, les cheveux et les poils. Notamment, la présente invention concerne la peau du visage, du décolleté, des bras et avant-bras, et des jambes, et le cuir chevelu. Plus particulièrement, le terme « annexe » désigne les cheveux, les poils et les ongles, et plus particulièrement les cheveux et les poils.

L'épiderme est conventionnellement divisé en une couche basale de kératinocytes constituant la couche germinative de l'épiderme, une couche dite épineuse pluristratifiée constituée de cellules polyédriques disposées sur la couche germinative, une à trois couches dites granuleuses constituées de cellules aplaties contenant des inclusions cytoplasmiques distinctes, les grains de kératohyaline, et enfin, un ensemble de couches supérieures appelées couches cornées, ou *stratum corneum,* constituées de kératinocytes au stade terminal de leur différentiation, appelés cornéocytes.

Les cornéocytes sont des cellules anucléées principalement constituées d'une matière fibreuse contenant des cytokératines, entourées d'une enveloppe cornée. Il y a en permanence production de nouveaux kératinocytes pour compenser la perte en continu de cellules épidermiques au niveau de la couche cornée, selon un mécanisme dénommé desquamation. L'ensemble du processus conduisant à la formation du *stratum corneum* et à son élimination fait l'objet d'une fine régulation sous le contrôle de multiples facteurs hormonaux ou cellulaires. Un déséquilibre de ces facteurs, s'il n'est pas rapidement régulé, finit par se traduire par un dysfonctionnement de la prolifération et/ou de la différenciation des cellules au niveau de la couche basale, ou de la desquamation.

De nombreux défauts ou désordres de la peau et de ses annexes peuvent être liés à une telle altération.

Ainsi, lorsque la prolifération et/ou la différenciation des cellules de la couche basale de l'épiderme sont accélérées par rapport à la desquamation, alors le *stratum corneum* tend à s'épaissir. Ce dysfonctionnement peut, selon son degré de manifestation, être associé à différents défauts esthétiques tels que des signes cutanés du vieillissement, un désordre de la fonction barrière de l'épiderme, ou des signes de sécheresse cutanée, ou, le cas échéant, à différents désordres pathologiques, comme par exemple, une hyperkératose, une xérose, une ichtyose, le psoriasis, ou des hyperkératoses réactionnelles.

A l'inverse, un ralentissement de la prolifération et/ou de la différenciation des cellules de la couche basale par rapport à la desquamation peut se manifester par un amincissement de l'épiderme, et plus particulièrement de la couche cornée. Ce dysfonctionnement peut, selon son degré de manifestation, être associé à différents défauts esthétiques tel qu'un défaut de cicatrisation, ou un défaut de ré-épithélialisation, notamment après un traitement de gommage cutané ou exfoliant, ou, le cas échéant, à différents désordres pathologiques, comme par exemple, des réactions d'origine immunitaire, généralement induites par un contact de la peau avec un ou plusieurs agents exogènes.

Différents déséquilibres dans les mécanismes hormonaux, cellulaires ou moléculaires sous jacents à la formation du *stratum corneum* peuvent être associés à ces désordres.

Par exemple, FR 2 842 209 décrit l'expression de la protéine SASPase dans l'épiderme, et sa mise en oeuvre comme cible dans le traitement de désordres cutanés. Toutefois, la détection de l'expression de la SASPase dans l'épiderme n'est qu'un élément de l'ensemble des mécanismes impliqués dans l'homéostasie de l'épiderme, et de nombreux aspects de ces mécanismes reste encore à déterminer.

Toulza et al. Genome Biology 2007, vol. 8, n°6 a par ailleurs identifié la Filaggrine 2 dans une étude du transcriptome de kératinocytes granuleux.

Ainsi, il demeure un besoin de déterminer plus précisément la nature et le rôle des facteurs impliqués dans l'homéostasie de l'épiderme, et en particulier dans la prolifération et/ou la différenciation des cellules de l'épiderme.

Notamment, il existe un besoin de déterminer la nature des agents susceptibles de moduler l'activité protéolytique de la SASPase.

Il existe encore un besoin d'identifier et de disposer de nouvelles cibles cosmétiques ou thérapeutiques à l'égard de la peau et/ou de ses annexes, et notamment impliquées dans la prolifération et/ou la différenciation des cellules de l'épiderme.

Il existe encore un besoin d'identifier et de disposer de nouveaux outils de criblage de composés actifs aptes à moduler la prolifération et/ou la différenciation des cellules de l'épiderme, qui soient à la fois rapides, sensibles, faciles à mettre en oeuvre et peu coûteux.

La présente invention a pour objet de satisfaire ces besoins.

Le présent texte décrit un peptide isolé, représenté par une séquence d'acides aminés choisie parmi SEQ ID NO: 9, SEQ ID NO: 16, un fragment de celle-ci, ou un homologue de celle-ci présentant une identité de séquence d'au moins 85 % et apte à modifier la conformation tridimensionnelle d'un complexe formé par interaction d'une première séquence d'acides aminés issue de la Filaggrine-2 avec une seconde séquence d'acides aminés issue de la SASPase.

Ainsi, selon un premier objet, la présente invention concerne un peptide isolé, représenté par une séquence d'acides aminés choisie parmi :

- SEQ ID NO: 9,
- un fragment de celle-ci choisi parmi les séquences SEQ ID NO : 10, SEQ ID NO : 11, SEQ ID NO : 12, SEQ ID NO : 13, SEQ ID NO : 14 et SEQ ID NO : 15, ou
- un homologue de celle-ci présentant une identité de séquence d'au moins 85 % et apte à se lier par affinité à une séquence d'acides aminés issue d'une SASPase.

Selon un mode de réalisation préféré, ledit peptide est codé par une séquence d'acides nucléiques représentée par une séquence choisie parmi SEQ ID NO : 1, un fragment de celle-ci, ou un homologue de celle-ci présentant une identité de séquence d'au moins 85%.

De manière inattendue, les inventeurs ont mis en évidence que la Filaggrine-2, sous forme de monomère ou de dimère, et la SASPase se lient l'une à l'autre par affinité pour former ensemble un complexe protéique. En particulier, les inventeurs ont identifié dans la Filaggrine-2 et la SASPase, les séquences précises d'acides aminés impliquées dans cette liaison et la formation du complexe protéique.

Qui plus est, les inventeurs ont mis en évidence que la liaison de la Filaggrine-2 à la SASPase augmente l'activité protéolytique de cette dernière, et que cette activité peut en outre être modulée par des peptides spécifiques issus de la Filaggrine-2.

Au sens de l'invention, on entend par « interaction », la réalisation d'une ou plusieurs liaisons par affinité entre deux séquences d'acides aminés de sorte que se forme un complexe protéique ou peptidique apte à demeurer stable dans des conditions physiologiquement acceptable. Dans la présente invention, les termes « interaction » et « association » sont utilisés indifféremment.

Au sens de l'invention, on entend par « conformation tridimensionnelle » d'un complexe, l'arrangement spatiale des séquences d'acides aminés composant ce complexe, prises ensembles, lorsqu'elles interagissent l'une avec l'autre.

Au sens de l'invention, par «modification» de la conformation tridimensionnelle d'un complexe, on entend une altération de l'arrangement spatial des séquences d'acides aminés dans le complexe ou une altération de la structure tridimensionnelle de l'une et/ou l'autre de ces séquences.

Ces altérations peuvent conduire à une modification de la forme d'ensemble, aussi nommée modification allostérique, du complexe et/ou à une modification de l'équilibre thermodynamique entre les séquences d'acides aminés libres et les séquences d'acides aminés liées entre elles. Ces altérations se traduisent par une augmentation, une diminution ou une stabilisation de l'activité biologique du complexe.

De manière préférée, ces altérations peuvent conduire à une dissociation d'un complexe, c'est-à-dire à un déplacement de l'équilibre thermodynamique établi entre les séquences libres et les séquences liées, vers les séquences libres.

Selon encore un autre aspect, la présente invention concerne un peptide chimère comprenant une première séquence d'acides aminés fusionnée à une seconde séquence d'acides aminés, dans lequel la première séquence d'acides aminés représente un peptide de l'invention.

Selon encore un autre aspect, la présente invention concerne un complexe isolé, formé par interaction d'une première séquence d'acides aminés avec une seconde séquence d'acides aminés issue d'une SASPase, ou d'un homologue de cette seconde séquence d'acides aminés présentant une identité de séquence d'au moins 85 % avec la séquence SEQ ID NO : 16, dans lequel ladite première séquence d'acides aminés représente un peptide de l'invention, notamment tel que défini selon le premier objet de l'invention.

Au sens de l'invention, les termes « séquence d'acides aminés issue de » visent à désigner tout ou partie, i.e. un fragment ou la totalité de la protéine ou du peptide considéré (e).

Selon encore un autre aspect, le présent texte décrit une utilisation d'au moins un complexe de l'invention pour cribler des composés aptes à réguler la prolifération et/ou la différenciation des cellules d'un épiderme.

Selon encore un autre aspect, le présent texte décrit un procédé de criblage d'un composé apte à réguler la prolifération et/ou la différenciation des cellules d'un épiderme, ledit procédé comprenant au moins les étapes décrites ci-après.

Un procédé ou une utilisation décrits dans le présent texte peuvent avantageusement être mis en oeuvre *in vivo, ex vivo* ou *in vitro.*

En particulier, un procédé ou une utilisation décrits peuvent être mis en oeuvre *ex vivo* ou *in vitro.*

Un procédé de criblage peut avantageusement être mis en oeuvre pour identifier de nouveaux composés actifs aptes à prévenir et/ou traiter un défaut esthétique ou un désordre pathologique de la peau et/ou de ses annexes lié à un dysfonctionnement de la différenciation et/ou de la prolifération des cellules de l'épiderme.

Ainsi, un autre objet de l'invention est un procédé de criblage *in vitro* ou *ex vivo* d'un composé étant un agoniste ou un antagoniste de l'interaction entre une première séquence d'acides aminés et une seconde séquence d'acides aminés présentant une identité de séquence d'au moins 85% avec la séquence SEQ ID NO : 16, dans lequel ladite première séquence d'acides aminés représente un peptide selon l'invention, ce composé étant également apte à réguler la prolifération et/ou la différentiation des cellules d'un épiderme, ledit procédé comprenant au moins les étapes consistant à :

a) disposer d'un complexe selon l'invention dans des conditions propices à sa formation,

b) associer, préalablement ou postérieurement à l'étape a), la première et/ou la seconde séquence(s) d'acides aminés à une entité,

la première et/ou la seconde séquence(s) d'acides aminés formant avec ladite entité un ensemble apte à émettre un signal après exposition à une longueur d'onde excitatrice, et la réalisation des étapes a) et b) conduisant à l'obtention dudit complexe,

c) soumettre ledit complexe à ladite longueur d'onde excitatrice pour obtenir un premier signal S1 caractéristique du complexe,

d) déterminer quantitativement ou qualitativement le signal S1,

e) mettre en contact le complexe avec un milieu présumé contenir un composé à cribler,

f) soumettre le milieu obtenu à l'étape e) à la longueur d'onde excitatrice pour obtenir un second signal S2,

g) déterminer quantitativement ou qualitativement le signal S2, et

h) comparer les premier et second signaux S1 et S2 pour tirer une conclusion relative à une éventuelle modification de la conformation tridimensionnelle du complexe en présence du composé criblé.

Au sens de la présente invention, on entend par « prévenir », le fait de réduire le risque ou la probabilité de survenue d'un phénomène donné, i.e. dans la présente invention, un défaut esthétique ou un désordre pathologique de la peau et/ou de ses annexes lié à un défaut de différenciation et/ou de prolifération des cellules de l'épiderme.

La présente invention permet, avantageusement, de mettre à disposition une nouvelle cible utile en cosmétique ou en thérapeutique à l'égard de la peau et/ou de ses annexes.

Avantageusement, les peptides de la présente invention peuvent être particulièrement utiles pour la mise en oeuvre d'un procédé de criblage de nouveaux actifs cosmétiques ou dermatologiques, qui soit simple, peu coûteux et sensible.

Avantageusement, la présente invention peut permettre d'identifier de nouveaux actifs capables d'agir sur une voie inédite de la régulation de l'homéostasie de l'épiderme et ainsi d'assurer un effet plus précis et une meilleure efficacité à l'égard de défauts cosmétiques ou de désordres pathologiques de la peau et/ou de ses annexes.

La présente invention permet avantageusement de disposer d'un nouvel outil de criblage particulièrement efficace et sensible pour la détection de nouveaux composés actifs utiles en cosmétique ou en thérapeutique à l'égard de la peau et/ou de ses annexes.

Ces composés actifs peuvent notamment être efficaces vis-à-vis des défauts esthétiques ou des désordres pathologiques de la peau et/ou de ses annexes liés à un défaut de prolifération et/ou de différenciation des cellules de l'épiderme.

Avantageusement, les peptides de la présente invention peuvent être également utiles à titre d'actif cosmétique.

## Peptides

Les peptides décrits peuvent être issus, ou dérivés, des séquences d'acides aminés de la SASPase ou de la FLG2, et sont aptes, dans des conditions appropriées, à modifier la conformation tridimensionnelle d'un complexe formé par interaction d'une première séquence d'acides aminés issue de la Filaggrine-2 avec une seconde séquence d'acides aminés issue de la SASPase.

En particulier, les peptides de l'invention peuvent être issus, ou dérivés, des séquences d'acides aminés de la FLG2, et sont aptes, dans des conditions appropriées, à modifier la conformation tridimensionnelle d'un complexe formé par interaction d'une première séquence d'acides aminés issue de la Filaggrine-2 avec une seconde séquence d'acides aminés issue de la SASPase.

Un tel complexe peut être formé par interaction de tout ou partie d'une première séquence d'acides aminés issue de la SASPase avec tout ou partie d'une seconde séquence d'acides aminés issue de la FLG2 ou un dimère de cette seconde séquence.

Les séquences d'acides aminés de la SASPase ou de la FLG2 convenant à un tel complexe peuvent, en particulier, être représentées par des séquences choisies parmi des séquences représentées par les références Q53RT3 et Q5D862 (Références Uniprot / Swissprot), des fragments de celles-ci, ou des homologues de celles-ci présentant une identité de séquence d'au moins 85 %, et une activité biologique de même nature.

Selon un mode de réalisation, un peptide de l'invention peut être apte à se lier par affinité à une séquence d'acides aminés issue de la SASPase. Une séquence d'acides aminés issue de la SASPase plus particulièrement considérée selon l'invention peut être la séquence d'acides aminés représentée par la séquence Q53RT3 (réf. Uniprot/Swissprot), et de préférence, la séquence d'acides aminés s'étendant des positions 85 à 343 de cette séquence. La séquence d'acides aminés s'étendant des positions 85 à 343 de la séquence Q53RT3 est présumée représenter une forme active de la SASPase comprenant 259 acides aminés, pour un poids moléculaire d'environ 28 kDa.

Un peptide de l'invention peut être issu de la FLG2. En particulier, une Filaggrine-2 convenant à l'invention peut être la Filaggrine-2 humaine, et plus particulièrement une Filaggrine-2 de séquence d'acides aminées représentée par la référence Q5D862 (réf. Uniprot/Swissprot). Cette séquence comprend 2391 acides aminés, et présente un poids moléculaire d'environ 948 kDa.

Selon une variante de réalisation préférée, l'invention concerne un peptide isolé, de séquence d'acides aminés représentée par la séquence SEQ ID NO: 9, un fragment de celle-ci choisi parmi les séquences SEQ ID NO : 10, SEQ ID NO : 11, SEQ ID NO : 12, SEQ ID NO : 13, SEQ ID NO : 14 et SEQ ID NO : 15, ou un homologue de celle-ci présentant une identité de séquence d'au moins 85 %. Un tel peptide est apte à se lier par affinité à une séquence d'acides aminés issue d'une SASPase.

La séquence d'acides aminés représentée par SEQ ID NO : 9 figure la séquence d'acides aminés s'étendant des positions 2 à 95 de la séquence de la référence Q5D862 de la Filaggrine-2.

Selon un mode de réalisation, un peptide de l'invention peut en particulier être constitué de 3 à 50 acides aminés, de préférence de 6 à 30 acides aminés, de préférence de 8 à 20 acides aminés, et de préférence encore de 8 à 16 acides aminés, voire encore de 10 à 16 acides aminés contigus issus de la séquence SEQ ID NO: 9.

De manière préférée encore, un peptide de l'invention peut en particulier être constitué de 3 à 10 acides aminés contigus issus de la séquence SEQ ID NO: 9. De tels peptides peuvent plus particulièrement convenir à un usage cosmétique, par exemple dans des compositions cosmétiques appliquées par voie topique ou transdermique.

Par ailleurs, un peptide décrit dans le présent texte peut être représenté par une séquence d'acides aminés choisie parmi SEQ ID NO: 10, SEQ ID NO: 11, SEQ ID NO: 12, SEQ ID NO: 13, SEQ ID NO: 14, SEQ ID NO: 15, un fragment de celle-ci, ou un homologue de celle-ci présentant une identité de séquence d'au moins 85 %.

En particulier, un peptide de l'invention peut être représenté par une séquence d'acides aminés choisie parmi SEQ ID NO: 10, SEQ ID NO: 11, SEQ ID NO: 12, SEQ ID NO: 13, SEQ ID NO: 14, SEQ ID NO: 15, ou un homologue de celle-ci présentant une identité de séquence d'au moins 85 %.

Ces peptides sont aptes à se lier par affinité à une séquence d'acides aminés issue d'une SASPase.

Selon un autre mode de réalisation, un peptide décrit dans le présent texte peut être apte à se lier par affinité à une séquence d'acides aminés issue de la FLG2. Une séquence d'acides aminés issue de la FLG2 plus particulièrement considérée peut être la séquence d'acides aminés représentée par la séquence Q5D862 (réf. Uniprot/Swissprot), et de préférence, la séquence d'acides aminés s'étendant des positions 2 à 213 de cette séquence.

Selon une variante de réalisation, un peptide décrit dans le présent texte peut être apte à se lier à un dimère d'une séquence d'acides aminés issue de FLG2.

Selon un autre mode de réalisation, un peptide décrit dans le présent texte apte à se lier par affinité à une séquence d'acides aminés issue de la FLG2 peut être issu de la SASPase. Une SASPase ou « Skin ASPartic protease » convenant à l'invention, peut être la SASPase humaine, et plus particulièrement, peut être une SASPase de séquence d'acides aminés représentés par la référence Q53RT3 (réf. Uniprot/Swissprot). Cette séquence comprend 343 acides aminés et présente un poids moléculaire d'environ 37 kDa.

Selon une variante de réalisation, le présent texte décrit un peptide isolé, de séquence d'acides aminés représenté par SEQ ID NO: 16, un fragment de celle-ci, ou un homologue de celle-ci présentant une identité de séquence d'au moins 85 %. De manière préférée, un tel peptide peut être apte à se lier par affinité à une séquence d'acides aminés issue d'une FLG2.

La séquence d'acides aminés représentée par SEQ ID NO : 16 figure la séquence d'acides aminés s'étendant des positions 97 à 173 de la séquence Q53RT3 de la SASPase (réf. Uniprot/Swissprot).

Selon un mode de réalisation, un peptide de l'invention apte à se lier par affinité à une séquence d'acides aminés issue d'une SASPase peut être codé par une séquence d'acides nucléiques représentée par une séquence choisie parmi SEQ ID NO: 1, un fragment de celle-ci, ou un homologue de celle-ci présentant une identité de séquence d'au moins 85 %.

De préférence, un peptide de l'invention apte à se lier par affinité à une séquence d'acides aminés issue d'une SASPase peut être codé par une séquence d'acides nucléiques représentée par SEQ ID NO: 1, SEQ ID NO: 2, SEQ ID NO: 3, SEQ ID NO: 4, SEQ ID NO: 5, SEQ ID NO: 6, SEQ ID NO: 7, un fragment de celle-ci, ou un homologue de celle-ci présentant une identité de séquence d'au moins 85 %.

Selon un mode de réalisation, un peptide apte à se lier par affinité à une séquence d'acides aminés issue d'une FLG2 peut être codé par une séquence d'acides nucléiques représentée par une séquence choisie parmi SEQ ID NO:

8, un fragment de celle-ci, ou un homologue de celle-ci présentant une identité de séquence d'au moins 85 %.

Selon un de ces aspects, le présent texte décrit également une séquence d'acides nucléiques isolée, codant pour un peptide de l'invention, un fragment de celle-ci, ou un homologue de celle-ci présentant une identité de séquence d'au moins 85 %, et en particulier telle que définie précédemment.

Une séquence d'acides nucléiques peut être mise en oeuvre pour la préparation de toutes séquences d'acides aminés de l'invention, et plus particulièrement pour la préparation de d'un peptide chimère comme indiqué ci-après.

Une séquence d'acides nucléiques selon le présent texte peut être issue de toute origine possible, à savoir soit animale, en particulier de mammifères, et encore plus particulièrement humaine, soit végétale, soit de micro-organismes, tels que des virus, des phages, des bactéries ou encore des champignons, sans préjuger du fait qu'elle soit ou non présente de manière naturelle dans ledit organisme.

Par « homologue » d'une séquence d'acides aminés ou d'une séquence d'acides nucléiques, on entend désigner une séquence présentant une identité d'au moins 85%, en particulier d'au moins 90%, en particulier d'au moins 98%, et plus particulièrement d'au moins 99 % avec ladite séquence, et possédant une activité biologique de même nature.

Une homologie de séquence peut être identifiée par toute technique habituellement utilisée dans le domaine par l'homme de l'art. A titre d'exemple, une homologie de séquence peut être déterminée par utilisation de l'interface informatique BLAST disponible sur le site Internet NCBI à l'adresse http://blast.ncbi.nlm.nih.gov configurée avec les paramètres par défaut.

Un homologue d'une séquence d'acides aminés peut différer de cette séquence, par exemple, par une ou plusieurs délétion(s), et/ou insertion(s) et/ou une ou plusieurs substitution(s) d'un acide aminé. De manière similaire, un homologue d'une séquence d'acides nucléiques peut différer de cette séquence, par exemple, par une ou plusieurs délétion(s), et/ou insertion(s) et/ou une ou plusieurs substitution(s) d'une base ou d'un codon. Ces modifications peuvent être combinées.

Selon une variante de réalisation, un homologue d'une séquence d'acides aminés ou d'acides nucléiques peut comprendre une ou plusieurs substitutions conservatrices d'acides aminés ou de codons.

Une substitution conservatrice est le remplacement, dans une séquence d'un acide aminé par un autre acide aminé doté de propriétés physico-chimiques sensiblement similaires, ou suffisamment proches de celles de l'acide aminé d'origine, pour que les propriétés et fonctions de la protéine ou du peptide ne soient pas, ou sensiblement pas, affectées. De même, une substitution conservatrice est le remplacement, dans une séquence d'acides nucléiques, d'un codon par un autre codon codant pour un acide aminé identique ou doté de propriétés physico-chimiques sensiblement similaires, ou suffisamment proches, de celles de l'acide aminé d'origine pour que les propriétés et fonctions de la séquence d'acides nucléiques codée ne soient pas ou sensiblement pas, affectées.

Les modifications des séquences d'acides aminés ou d'acides nucléiques présentées ci-dessus peuvent être dénommées, de manière générale, « mutation ». Ainsi, les homologues de l'invention concernent également les mutants et les variants des séquences d'acides aminés ou d'acides nucléiques de l'invention.

Selon un mode de réalisation, une séquence d'acides aminés issue de la SASPase peut comprendre une ou plusieurs mutations.

A titre d'exemple de mutations de la SASPase pouvant être considérées dans la présente invention, on peut mentionner le remplacement d'un ou plusieurs résidus d'acides aminés par des résidus d'acides aminés ayant un indice hydropathique similaire sans pour autant affecter de manière sensible les propriétés biologiques de la SASPase, et notamment sa propriété d'interaction avec son peptide partenaire. L'indice hydropathique est un indice attribué aux acides aminés en fonction de leur hydrophobicité et de leur charge (Kyte & Doolittle, J. Mol. Biol., 1982, 157:105).

Une séquence d'acides aminés convenant à l'invention peut comprendre une ou plusieurs modification(s) post-traductionnelle(s).

Par "modification(s) post-traductionnelle(s)", on entend englober l'ensemble des modifications qu'une séquence d'acides aminés est susceptible de subir à l'issu de sa synthèse dans une cellule, telle(s) que par exemple une ou des phosphorylation(s), une ou des glycosylation(s), une ou des citrullinations, une ou des lipidation(s), telles qu'une farnésylation ou une palmitoylation, un réarrangement structural de type formation de ponts disulfures et/ou clivage à l'intérieur de la séquence peptidique.

Par « activité biologique de même nature » d'une séquence d'acides aminés de l'invention, on entend en particulier sa capacité à se lier par affinité avec une séquence d'acides aminés partenaire. Par « activité biologique de même nature » d'une séquence d'acides nucléiques, on entend en particulier sa capacité à être transcrite et traduite en une séquence d'acides aminés.

Selon une variante de réalisation, à l'égard de la SASPase, une activité biologique de même nature peut s'entendre, également, de son activité protéolytique, notamment à l'égard de la cornéodesmosine.

Selon une autre variante de réalisation de l'invention, lors de la mise en oeuvre d'un mutant inactif de la SASPase, on entend par « activité biologique de même nature », principalement, la propriété du mutant à se lier par affinité à une séquence d'acides aminés de l'invention issue de FLG2.

Ainsi, une séquence d'acides aminés de la SASPase convenant à l'invention est nécessairement doté au moins de la propriété d'interagir avec une séquence d'acides aminés de la FLG2.

A l'égard d'une séquence d'acides aminés issue de FLG2, une activité biologique de même nature peut s'entendre, en outre, de sa propriété à favoriser et/ou augmenter l'activité protéolytique de la SASPase.

Selon une autre variante de l'invention, une activité biologique de même nature peut en outre s'entendre de l'activité biologique d'un complexe de l'invention à l'égard de la prolifération et/ou de la différenciation des cellules de l'épiderme.

Au sens de l'invention; on entend par « fragment » d'une séquence d'acides aminés toute portion issue de cette séquence et constituée de 3 à 50 acides aminés, de préférence de 6 à 30 acides aminés, de préférence de 8 à 20 acides aminés, et de préférence encore de 10 à 16 acides aminés contigus, et dotée d'une activité biologique de même nature que celle exprimée par la séquence d'acides aminés.

Le terme « fragment » à l'égard d'une séquence d'acides nucléiques désigne toute portion de cette séquence, et constituée de 9 à 150 bases, de préférence de 18 à 90 bases, de préférence de 24 à 60 bases, et de préférence encore de 30 à 48 bases, et codant pour un fragment de la séquence d'acides aminés codée ladite séquence.

Selon un mode de réalisation, une séquence d'acides aminés de l'invention peut être une séquence d'acides aminés naturel(le) ou synthétique, le cas échéant susceptible d'être obtenu(e) par synthèse chimique ou biologique, ou par extraction à partir d'un tissu biologique, comme par exemple la peau, exprimant naturellement ou après transduction une séquence d'acides aminés, ainsi que les différentes formes post-traductionnelles de celle-ci.

Un peptide de l'invention peut également être choisi parmi des peptidomimétiques. Un peptidomimétique de l'invention peut être obtenu par modification d'une séquence d'acides aminés de l'invention, ou par synthèse d'un composé mimant une telle séquence, tel qu'un peptoïde ou un β-peptide. Les modifications considérées peuvent se fonder sur l'introduction de changements dits « non-naturels » dans la séquence d'acides aminés, tels que des modifications du squelette ou l'intégration d'acides aminés non-naturels.

Le présent texte décrit également un peptide chimère comprenant une première séquence d'acides aminés fusionnée ou couplée avec une seconde séquence d'acides aminés, un agent de ciblage hydrophile ou hydrophobe, un précurseur de bioconversion, un marqueur d'affinité par exemple un marqueur fluorescent, un marqueur luminescent, un marqueur radioactif ou un marqueur colorimétrique.

De manière non limitative, on peut citer comme exemple de composés susceptibles d'être couplés avec une séquence d'acides aminés de l'invention, des protéines fluorescentes comme la "Green Fluorescent Protein", des composés chimiques fluorescents tels que la rhodamine, la fluorescéine, ou le Texas Red, des composés phosphorescents, des éléments radioactifs, tels que $^{3}$H, $^{35}$S, $^{99}$Tc, $^{121}$I ou $^{125}$I, des marqueurs colorimétriques comme les substrats chromogènes sensibles à l'action de la galactosidase, de la peroxydase, de la chloramphénicol acétyltransférase, de la luciférase ou de la phosphatase alcaline, ou encore des marqueurs d'affinité, notamment de type His-tag, GST, ou MBP.

Selon la nature des composés susceptibles d'être couplés avec une séquence d'acides aminés de l'invention, le couplage peut être opéré par tout procédé chimique ou tout procédé de biologie moléculaire connu de l'homme de l'art. Dans le dernier cas, on peut parler de peptide ou de protéine de fusion, ou de peptide ou protéine chimère.

Ainsi, la présente invention a également pour objet un peptide chimère comprenant une première séquence d'acides aminés fusionnée à une seconde séquence d'acides aminés, dans lequel la première séquence d'acides aminés représente un peptide de l'invention tel que défini précédemment.

Selon un mode de réalisation un peptide chimère selon l'invention peut comprendre à titre de seconde séquence d'acides aminés, une séquence choisie parmi une protéine fluorescente, un marqueur d'affinité, un peptide signal, un peptide pro-pénétrant, et de préférence est un marqueur d'affinité.

Selon un mode de réalisation, une première séquence d'acides aminés d'un peptide chimère peut être une séquence d'acides aminés issue d'une FLG2 ou d'une SASPase, et plus préférentiellement des séquences SEQ ID NO: 9 ou SEQ ID NO: 16.

Selon un mode de réalisation, une première séquence d'acides aminés d'un peptide chimère de l'invention est une séquence d'acides aminés issue d'une FLG2, et plus préférentiellement de la séquence SEQ ID NO: 9.

A titre de seconde séquence d'acides aminés préférée d'un peptide chimère de l'invention on peut citer les protéines fluorescentes, et en particulier la GFP (Green Fluorescent Protein), l'ECFP (Enhanced Cyan Fluorescent Protein), la DsRed2FP (DsRed fluorescent protein), l'EGFP (Enhanced Green Fluorescent Protein), l'EYFP (Enhanced Yellow Fluorescent Protein), l'EBFP (Enhanced Blue Fluorescent Protein).

A titre de marqueur d'affinité convenant à l'invention, on peut citer en particulier la glutathion-S-transférase (GST), la protéine liant le maltose (maltose binding protein, MBP), la thiorédoxine (THX), un marqueur Myc, un marqueur FLAF, un marqueur His, un marqueur THX-His-tag ou THX-His-S-tag.

Un peptide chimère de l'invention peut être obtenu par tout procédé de biologie moléculaire connu de l'homme de l'art, notamment comme décrit par SAMBROOK et al. (Molecular Cloning : A laboratory Manual, Ed. Cold Spring Harbor, 2001).

Selon un autre de ses objets, l'invention concerne un complexe isolé, formé par interaction d'une première séquence d'acides aminés avec une seconde séquence d'acides aminés issue d'une SASPase, ou d'un homologue de celle-ci présentant une identité de séquence d'au moins 85%, dans lequel ladite première séquence d'acides aminés représente un peptide issu de FLG2, et de préférence tel que défini précédemment.

Selon une variante de réalisation préférée, une seconde séquence d'acides aminés d'un complexe de l'invention peut représenter un peptide issu d'une SASPase tel que défini précédemment.

**Criblage**

Le présent texte décrit l'utilisation d'au moins un complexe de l'invention pour cribler des composés aptes à réguler la prolifération et/ou la différenciation des cellules d'un épiderme.

Selon un mode de réalisation, l'invention concerne un procédé de criblage d'un composé apte à réguler la prolifération et/ou la différenciation des cellules d'un épiderme, tel que défini précédemment.

De préférence, un tel composé peut moduler la conformation tridimensionnelle d'un complexe formé par interaction d'une première séquence d'acides aminés issu de FLG2, ou d'un dimère d'une telle séquence, avec une seconde séquence d'acides aminés issu de la SASPase.

De préférence encore, un tel composé peut moduler la formation d'un complexe formé par interaction d'une première séquence d'acides aminés issu de FLG2, ou d'un dimère d'une telle séquence, avec une seconde séquence d'acides aminés issu de la SASPase.

Avantageusement, les séquences d'acides aminés de la FLG2 et de la SASPase peuvent être telles que définies précédemment.

Selon une variante de réalisation, un complexe considéré peut être un complexe de l'invention tel que défini précédemment.

Un procédé de l'invention peut comprendre au moins les étapes consistant à :

a) disposer d'un complexe de l'invention dans des conditions propices à sa formation,

b) associer, préalablement ou postérieurement à l'étape a), la première et/ou la seconde séquence(s) d'acides aminés à une entité,

la première et/ou la seconde séquence(s) d'acides aminés formant avec ladite entité un ensemble apte à émettre un signal après exposition à une longueur d'onde excitatrice, et la réalisation des étapes a) et b) conduisant à l'obtention dudit complexe,

c) soumettre ledit complexe à ladite longueur d'onde excitatrice pour obtenir un premier signal S1 caractéristique du complexe,

d) déterminer quantitativement ou qualitativement le signal S1,

e) mettre en contact le complexe avec un milieu présumé contenir un composé à cribler,

f) soumettre le milieu obtenu à l'étape e) à la longueur d'onde excitatrice pour obtenir un second signal S2,

g) déterminer quantitativement ou qualitativement le signal S2, et

h) comparer les premier et second signaux S1 et S2 pour tirer une conclusion relative à une éventuelle modification de la conformation tridimensionnelle du complexe en présence du composé criblé.

Selon un mode de réalisation, une entité convenant à l'invention peut être choisie parmi un support convenant à la résonance plasmonique de surface, un marqueur fluorescent, un anticorps ou une combinaison d'anticorps primaire et secondaire, ledit anticorps ou ledit anticorps secondaire portant un marqueur fluorescent.

Une première et/ou une seconde séquences d'acides aminés de l'invention peuvent être associées à une entité par toute méthode connue de l'homme de l'art, et adaptée à la nature du support à la première et/ou la seconde d'acides aminés doivent être associés. Une méthode de couplage sera naturellement choisie de sorte à ne pas affecter les propriétés de liaison ou d'affinité des séquences d'acides aminés utilisées.

Selon un mode de réalisation, une association entre une première et/ou une seconde séquences d'acides aminés peut être effectuée par couplage chimique, par biologie moléculaire, ou par interaction(s) spécifique(s) de forte affinité.

En particulier, une association peut être opérée par un couplage chimique, notamment au moyen de fonctions chimiques réactives, ou par un procédé de biologie moléculaire.

Lors de la mise en oeuvre d'une association par procédé chimique, les fonctions chimiques à faire réagir peuvent être naturellement présentes sur la première et/ou la seconde séquences d'acides aminés, ou peuvent être introduites sur la première et/ou la seconde séquences d'acides aminés et l'entité préalablement à la réaction chimique.

A titre d'exemple de fonctions chimiques réactives convenant à l'invention, on peut par exemple mentionner celles classiquement utilisées dans les réactions dites de « chimie click » (click chemistry).

A titre d'exemple de procédé de biologie moléculaire convenant à l'association entre une entité et la première et/ou la seconde séquences d'acides aminés, on peut citer ceux classiquement mis en oeuvre dans les procédés de clonage comprenant, notamment, la préparation de séquences d'acides nucléiques appropriées, leur ligation et leur introduction dans un vecteur d'expression adapté, par exemple un plasmide, et la transfection du plasmide dans une cellule hôte apte à permettre l'expression et l'obtention d'un peptide chimère de l'invention.

Selon un autre mode de réalisation, une entité et la première et/ou la seconde séquences d'acides aminés peuvent être associé(e)s l'un(e) à l'autre au moyen d'une ou d'interaction(s) spécifique(s) de forte(s) affinité(s).

Selon un mode de réalisation, une entité convenant en particulier à l'invention peut être un anticorps ou un ensemble d'anticorps primaire et secondaire.

Un anticorps convenant à l'invention peut être obtenu par tout procédé connu de l'homme de l'art, notamment comme décrit dans « Antibodies : A Laboratory Manual », Cold Spring Harbor Laboratory Press, Cold Spring Harbor, N.Y. (1990).

A titre de type d'anticorps convenant à l'invention, on peut citer les anticorps monoclonaux ou polyclonaux, ainsi que des fragments d'immunoglobuline susceptibles de lier un antigène et qui peuvent être produits par toute technique de génie génétique connue de l'homme de l'art ou par coupure enzymatique ou chimique d'anticorps entiers.

Plus particulièrement, un anticorps convenant à l'invention peut être choisi parmi un anticorps monoclonal, un anticorps polyclonal, un diabodie, un anticorps scFv, un anticorps F(ab')$^2$, un anticorps Fab', un anticorps chimérique, un anticorps humanisé, et un anticorps recombinant.

Selon un mode de réalisation, un anticorps convenant à l'invention peut présenter une affinité pour une partie de la première et/ou de la seconde séquences d'acides aminés contre laquelle il est dirigé, ou pour un marqueur d'affinité spécifique porté la première et/ou la seconde séquences d'acides aminés.

Selon un mode de réalisation de la présente invention la première et/ou la seconde séquences d'acides aminés peuvent porter un marqueur d'affinité reconnu par un anticorps.

Un marqueur d'affinité convenant à l'invention peut notamment être choisi parmi les marqueurs d'affinité précédemment indiqués.

A titre d'exemples d'anticorps convenant à l'invention, on peut en particulier citer ceux mis en oeuvre dans les exemples exposés ci-après.

Selon un autre mode de réalisation, la première et/ou la seconde séquences d'acides aminés sont associées, respectivement, à une première et une seconde entités, lesdites entités comportant, respectivement, un groupe fluorescent A et un groupe fluorescent B, lesdits groupes A et B définissant un couple accepteur-donneur d'énergie de fluorescence convenant à la mise en oeuvre d'un transfert d'énergie par résonance de fluorescence.

A titre d'exemples de couples accepteur-donneur utilisables selon la présente invention, on peut citer, de façon non limitative, les couples BFP (Blue Fluorescent Protein)/GFP (Green Fluorescent Protein), CFP (Cyan Fluorescent Protein)/YFP (Yellow Fluorescent Protein), CFP/DsRed (Red Fluorescent Protein), GFP/DsRed et EuK (Cryptate d'Europium)/XL665. De préférence, un couple accepteur-donneur convenant à l'invention peut être EuK/XL665.

Selon une variante de réalisation, les première et seconde entités peuvent être des marqueurs fluorescents. De tels marqueurs fluorescents peuvent être choisis parmi des molécules fluorescentes ou des protéines fluorescentes habituellement mise en oeuvre dans le domaine. De manière préférée, les marqueurs fluorescents utilisés peuvent être choisis parmi des protéines fluorescentes fusionnées par biologie moléculaire, notamment comme indiqué précédemment, à la première et/ou à la seconde séquence d'acides aminés.

Selon une autre variante de réalisation, les première et seconde entités peuvent être des anticorps portant des marqueurs fluorescents et peuvent être aptes à se fixer, respectivement, sur un premier et un second marqueur d'affinité portés, respectivement, par la première et/ou la seconde séquence d'acides aminés.

Selon un mode de réalisation, le signal S1 déterminé quantitativement ou qualitativement à l'étape d) du procédé selon l'invention peut être un signal fluorescent obtenu par irradiation à une longueur d'onde excitatrice du donneur d'énergie de fluorescence.

Le signal S2, déterminé quantitativement ou qualitativement à l'étape g) du procédé, peut être un signal fluorescent obtenu par irradiation à une longueur d'onde excitatrice du donneur d'énergie de fluorescence.

Les signaux de fluorescence S1 et S2 peuvent être mesurés à l'aide de tout dispositif adapté à l'invention et connu de l'homme de l'art. A titre d'exemples de dispositifs convenant à l'invention, on peut citer un lecteur de microplaques, un microscope à fluorescence, un spectrofluoromètre, ou un scanner à fluorescence.

Selon un mode de réalisation préféré, un procédé de l'invention peut être effectué en haut-débit, dans des microplaques, et les signaux de fluorescence peuvent être mesurés au moyen d'un lecteur de microplaques, par exemple un lecteur Pherastar (BMG).

Un signal S2 significativement plus grand que le signal S1 en présence d'un composé à cribler, peut être indicatif d'un composé favorisant l'équilibre et la stabilité de la conformation tridimensionnelle d'un complexe de l'invention

De manière préférée, un tel composé peut favoriser, ou être agoniste, de l'interaction la première et la seconde séquences d'acides aminés.

En revanche, un signal S2 significativement plus petit que le signal S1 en présence d'un composé à cribler, peut être indicatif d'un composé déstabilisant ou alternant la conformation tridimensionnelle d'un complexe de l'invention.

De manière préférée, un tel composé peut déstabiliser, ou être antagoniste, de l'interaction entre la première et la seconde séquence d'acides aminés.

Un procédé selon l'invention permet le criblage d'agent modulateur de la prolifération et/ou la différenciation des cellules de l'épiderme.

Au sens de l'invention, on entend par « composé modulateur » tout composé susceptible, ou apte, à agir, directement ou indirectement, sur un complexe comprenant une association d'une première séquence d'acides aminés issue de FLG2, ou d'un dimère de cette séquence, avec une seconde séquences d'acides aminés issue de la SASPase en vue de moduler la conformation tridimensionnelle de cette association.

Selon un mode de réalisation de l'invention, un composé criblé peut être un agoniste ou un antagoniste de cette association.

Selon une variante de réalisation, un composé modulateur peut favoriser, ou être agoniste, de cette association. Alternativement, selon une autre variante de réalisation, un composé modulateur peut être un composé déstabilisant, ou antagoniste, de cette association.

La nature agoniste ou antagoniste d'un composé modulateur à l'égard de cette association sera plus particulièrement retenue selon l'effet modulateur à exercer sur la prolifération et/ou la différenciation des cellules de l'épiderme.

Ainsi, selon la nature du défaut esthétique ou du désordre pathologique de la peau et/ou de ses annexes à prévenir ou à traiter, on mettra en oeuvre plus particulièrement un composé modulateur agoniste ou antagoniste de cette association.

Selon un mode de réalisation préféré de l'invention, un composé criblé peut être apte à prévenir et/ou traiter un défaut esthétique ou un désordre pathologique de la peau et/ou de ses annexes lié à un dysfonctionnement de la différentiation et/ou de la prolifération des cellules de l'épiderme.

Selon un mode de réalisation, un procédé de criblage de l'invention peut comprendre en outre au moins une étape supplémentaire consistant à évaluer la propriété d'un composé criblé à moduler la différentiation et/ou la prolifération des cellules d'un épiderme.

Une telle évaluation peut être effectuée *in vitro* ou *ex vivo,* notamment sur des cellules d'épiderme en culture. Les cellules peuvent être des cellules en lignées, c'est-à-dire transformées par un virus ou d'origine tumorale pour les immortaliser.

Alternativement, les cellules peuvent être des cellules de culture primaire, c'est-à-dire issues d'un tissu prélevé chez un organisme vivant, par exemple un épiderme d'un être humain.

De préférence, les cellules en culture peuvent être des fibroblastes, voire des kératinocytes, notamment stratifiés. Les cultures de cellules peuvent être effectuées selon toute méthode connue de l'homme de l'art.

Egalement, une évaluation *in vitro* peut être effectuée sur un modèle d'épiderme reconstruit. De tels modèles d'épiderme peuvent être disponibles commercialement, par exemple comme le modèle Episkin®.

L'évaluation *in* vitro ou *ex* vivo d'un composé criblé selon l'invention peut être effectuée par tout protocole connu de l'homme de l'art visant à comparer l'effet donné d'un composé à tester à une valeur contrôle ou standard.

L'effet d'un composé criblé selon l'invention, *in vitro* ou *ex vivo*, peut être déterminé par la mesure de marqueurs biologiques connus comme étant spécifiquement associés à la différentiation et/ou la prolifération des cellules d'un épiderme.

De tels marqueurs peuvent être choisis, par exemple, parmi la mesure de l'épaisseur du *stratum corneum,* de sa fonction barrière, la mesure de l'expression et/ou de l'activation de la transglutaminase I, de la Filaggrine, de la Caspase 14, de la SASPase, de la Cornéodesmosine, de la Desmogléine ou de la protéine Ki67.

Ces marqueurs peuvent être déterminés, par exemple, par Western blot ou immunofluorescence selon tout protocole connu de l'homme de l'art.

### Utilisations et compositions

Un composé criblé selon l'invention, un peptide selon l'invention ou une séquence d'acides nucléiques décrite dans le présent texte peuvent être utilisé en cosmétique à titre d'agent actif cosmétique pour prévenir et/ou traiter un défaut esthétique de la peau et/ou de ses annexes lié à un dysfonctionnement de différentiation et/ou de prolifération des cellules d'un épiderme.

Un composé criblé selon l'invention, un peptide selon l'invention ou une séquence d'acides nucléiques décrite dans le présent texte peuvent être utilisé pour la préparation d'une composition thérapeutique destinée à prévenir et/ou à traiter un désordre pathologique de la peau et/ou de ses annexes lié à un dysfonctionnement de différentiation et/ou de la prolifération des cellules d'un épiderme.

Egalement, le présent texte décrit une composition thérapeutique comprenant à titre d'actif un composé criblé selon l'invention, un peptide selon l'invention ou une séquence d'acides nucléiques décrite dans le présent texte pour prévenir et/ou traiter un désordre pathologique de la peau et/ou de ses annexes lié à un dysfonctionnement de différentiation et/ou de prolifération des cellules d'un épiderme.

L'application cosmétique ou thérapeutique d'un composé criblé selon l'invention, un peptide selon l'invention ou une séquence d'acides nucléiques décrite dans le présent texte dépend de la nature, voire du degré ou de l'intensité de manifestation des signes exprimés par la peau et/ou ses annexes consécutivement à un dysfonctionnement de différentiation et/ou de prolifération des cellules d'un épiderme. Il relève des connaissances générales de l'homme de l'art d'apprécier cette nature ou cette intensité de manifestation et mettre en oeuvre l'application adéquate.

Le présent texte décrit l'utilisation cosmétique d'un peptide de l'invention ou d'une séquence acides nucléiques codant pour un tel peptide à titre d'actif cosmétique pour prévenir et/ou traiter un défaut esthétique de la peau et/ou de ses annexes lié à un dysfonctionnement de la différenciation et/ou de la prolifération des cellules d'un épiderme.

Un défaut esthétique considéré par l'invention peut être choisi parmi des signes cutanés du vieillissement, un désordre de la fonction barrière de l'épiderme, des signes de sécheresse cutanée, des signes cutanés d'une hyperséborrhée, une altération du teint de la peau, un désordre de la desquamation de la peau ou du cuir chevelu, un défaut de cicatrisation, et/ou de ré-épithélialisation.

Un défaut esthétique considéré par l'invention peut également être une hypersudation et/ou un problème d'odeur corporelle.

Selon un autre mode de réalisation, un peptide selon l'invention ou une séquence d'acides nucléiques décrite dans le présent texte peuvent également être utilisés en cosmétique à titre d'agent actif pour favoriser la ré-épithélialisation après un traitement de gommage cutané ou exfoliant.

Selon un autre mode de réalisation, un peptide selon l'invention ou une séquence d'acides nucléiques décrite dans le présent texte peuvent être utilisés en cosmétique à titre d'agent actif pour prévenir et/ou traiter la chute des cheveux et/ou favoriser la repousse des cheveux, ou prévenir la repousse des poils.

Selon un mode de réalisation, un désordre pathologique peut être choisi parmi le cancer, le psoriasis, le vitiligo, la rosacée, la dermatite atopique, la dermatite séborrhéique et l'acné.

Selon un mode de réalisation, une utilisation cosmétique du présent texte peut mettre en oeuvre de manière préférée un peptide de l'invention issu de la FLG2 et apte à se lier par affinité à une séquence d'acides aminés issue d'une SASPase ou d'une séquence d'acides nucléiques codant pour un tel peptide, notamment tels que définis précédemment, à titre d'actif cosmétique pour prévenir et/ou traiter le vieillissement d'un épiderme et/ou améliorer la fonction barrière d'un épiderme.

Selon un mode de réalisation, une utilisation cosmétique du présent texte peut mettre en oeuvre de manière préférée un peptide de l'invention issu de la SASPase et apte à se lier par affinité à une séquence d'acides aminés issue d'une Filaggrime 2, notamment tels que définis précédemment, à titre d'actif cosmétique pour prévenir et/ou traiter le vieillissement d'un épiderme et/ou améliorer la fonction barrière d'un épiderme.

Selon un de ses aspects, l'invention concerne une composition cosmétique comprenant dans un milieu physiologique acceptable une quantité efficace d'au moins un peptide de l'invention ou d'une séquence d'acides nucléiques codant pour un tel peptide.

Le présent texte décrit une composition cosmétique ou thérapeutique comprenant une quantité efficace d'au moins un peptide de l'invention ou une séquence d'acides nucléiques codant pour un tel peptide, à titre d'agent actif pour traiter et/ou prévenir un défaut esthétique ou un désordre pathologique de la peau et/ou de ses annexes, lié à un dysfonctionnement de la prolifération et/ou de la différenciation des cellules de l'épiderme.

Au sens de la présente invention, on entend par « quantité efficace », une quantité suffisante et nécessaire d'un actif pour exercer un effet de prévention et/ou de traitement à l'égard d'un défaut esthétique ou un désordre pathologique de la peau et/ou de ses annexes.

Une telle composition peut être administrée par voie orale, parentérale ou topique.

De telles voies de mise en oeuvre sont connues de l'homme de l'art.

Selon un autre de ces aspects, la présente invention concerne une composition comprenant au moins une séquence d'acides nucléiques codant pour un peptide de l'invention.

Une telle composition peut être dédiée à permettre l'expression d'un peptide de l'invention dans une cellule, un tissu biologique ou un organe.

Une telle composition peut être mise en oeuvre à des fins cosmétiques, thérapeutiques ou diagnostiques.

Selon un autre aspect, le présent texte décrit un procédé de traitement cosmétique pour prévenir et/ou traiter un défaut esthétique de la peau et/ou de ses annexes lié à un dysfonctionnement de la différentiation et/ou de la prolifération des cellules de l'épiderme chez un individu, comprenant au moins une étape d'administration audit individu à titre d'actif d'une quantité efficace d'au moins un peptide ou d'une séquence d'acides nucléiques de l'invention.

Un tel procédé peut avantageusement être mis en oeuvre par voie topique, orale ou parentérale, et en particulier par voie topique ou orale.

La voie parentérale comprend en particulier la voie intra-dermique et la voie sous-cutanée.

Selon un autre aspect, le présent texte décrit l'utilisation d'une quantité efficace d'au moins un peptide de l'invention ou d'au moins une séquence d'acides nucléiques décrite dans le présent texte ou d'au moins un composé criblé de l'invention pour la préparation et/ou l'amélioration d'un modèle cellulaire pluristratifié, notamment de type épidermique ou muqueux, et en particulier un modèle de peau reconstruite.

Avantageusement, un peptide selon l'invention ou une séquence d'acides nucléiques décrite dans le présent texte ou un composé criblé de l'invention sont ajoutés au milieu de culture dans lequel le modèle de culture pluristratifié est cultivé. L'étape de culture et la durée d'incubation du modèle cellulaire effectuées en présence d'une séquence d'acides aminés selon l'invention ou d'acides nucléiques décrite dans le présent texte ou d'un composé criblé de l'invention sont naturellement adaptées par l'homme de l'art en fonction du modèle cellulaire à obtenir et de l'effet à obtenir.

Au sens de l'invention, on entend désigner par « modèle de peau reconstruite », un modèle dans lequel sont associés différents types cellulaires, tels que notamment les cellules naturellement présentes dans une peau, à l'image par exemple des kératinocytes, des fibroblastes, des cellules de Langerhans et des mélanocytes. Les cellules de type fibroblastes peuvent être irradiées ou non.

De tels modèles et leur préparation sont connus de l'homme de l'art.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

Les exemples figurant ci-après sont présentés à titre illustratif et non limitatif de l'invention.

## EXEMPLES

### Exemple 1

*Interaction SASPase-FLG2 et identification des domaines peptidiques impliqués dans l'interaction*

Une première étude utilisant la technique du « double-hybride » a été réalisée en utilisant la protéine SASPase 28kDa entière, sous sa forme sauvage (SASPase_28, Réf. GENBANK gi16553767) et sous sa forme mutée au niveau de l'acide aspartique catalytique (SASPase_28$_{D128A}$), ainsi que la forme SASPase 37kDa (SASPase_37), sous sa forme entière (Réf. GENBANK gi189409121), et un fragment correspondant à l'extrémité N-terminale de 84 acides aminés de la protéine par rapport à la séquence de la SASPase_28 (SASPase_27$_{N84}$).

Les séquences de la SASPase ont été fusionnées à un domaine de liaison de l'ADN, LexA, selon l'orientation N-LexA-SASPase-C, pour conserver l'extrémité C-terminale de la SASPase libre, à l'exception de la séquence SASPase_28$_{N84}$ qui est fusionné au domaine LexA selon l'orientation N-SASPase_28$_{N84}$-LexA-C.

Les séquences proies ont été fusionnées au domaine activateur de transcription Gal4.

La SASPase_28, sauvage ou mutée, la SASPase_37 et la SASPase__28$_{N84}$ n'ont pas présenté d'autoactivation du système double-hybride. Les cribles réalisés avec les séquences de la SASPase_28 ou de la SASPase_37 ont donc permis d'identifier une interaction avec la Filaggrine 2 (FLG2), avec un très bon score de confiance.

L'interaction avec la SASPase a été retrouvée en utilisant comme appât le fragment de la Filaggrine 2 identifié comme interagissant avec les séquences de la SASPase_28 et de la SASPase_37, et correspondant aux acides aminés 2-213 la séquence GENBANK : gi:62122916.

La séquence a été fusionnée au domaine de liaison de l'ADN, LexA, selon l'orientation N-Lex A-FLG2$_{2-213}$-C.

Les séquences proies ont été fusionnées au domaine activateur de transcription Gal4.

Le fragment N-terminal de la Filaggrine 2 a présenté une légère autoactivation du système double-hybride, et une concentration de 5 mM de 3-aminotriazole a donc été utilisée pour le crible.

Le crible a permis de confirmer l'interaction avec la SASPase.

Des expériences complémentaires ont été réalisées afin de réduire et d'identifier la taille des séquences responsables de cette interaction entre la SASPase 28 et la Filaggrine 2.

Ces expériences ont permis de montrer qu'un fragment N-terminal de la SASPase 28 (acides aminés 11-86, SEQ ID NO: 16) était suffisant pour l'interaction avec FLG2.

A l'égard de FLG2, ces expériences complémentaire ont montré que le domaine S100 N-terminal de la filaggrine 2 (acides aminés 2-95, SEQ ID NO: 9) est impliqué dans l'interaction avec la SASPase.

### Exemple 2

*Modulation de l'activité protéolytique de la SASPase par FLG2*

Une Filaggrine-2 (FLG2) recombinante, constituée des acides aminés s'étendant des positions 2 à 213 de la séquence représentée par la référence Q5D862 (Réf. : Uniprot/Swissprot) fusionnée à un marqueur d'affinité Thioredoxine-His-S.Tag (THX-His-S-tag) a été préparée en plasmide parental pET32c (Novagen) (FLG2$_{2-213}$-Thioredoxine-His-S.Tag ou FLG2-HS).

Une SASPase recombinante de 28 kDa (SASPase_28) constituée des acides aminés s'étendant des positions 85 à 343 de la séquence représentée par la référence Q53RT3 (Réf. Uniprot/Swissprot) a été préparée comme décrit par BERNARD et al. (J. Invest. Dermatol., 2005, 125 : 278-287).

La SASPase_28 à 0,025 mg/ml est en présence d'un substrat colorimétrique Dabcyl-QIDRIMEK-Edans à 0,01 mM (Production Jerini Peptide Réf. D17 : JPT Peptide Technologies GmbH) dans un tampon PBS.

FLG2-Hs est mise en oeuvre à 0,01, 0,02, 0,04, 0,08, 0,16, 0,31, 0,63, 1,25, 2,5, 5 ou 10 fois la concentration finale de SASPase (0,025 mg/ml).

L'hydrolyse du substrat colorimétrique par la SASPase activée conduit à la séparation du fluorochrome EDANS et de son désactivateur (ou quencheur) Dabcyl, et à un accroissement de la fluorescence de EDANS.

La mesure du signal fluorescent est effectuée à $\lambda_{ex}$ 340 nm/$\lambda_{em}$ 490 nm. La fluorescence est lue toutes les 10 minutes pendant lh30, à 37°C, avec un SPECTRAMAX M5e (Molecular Devices).

Les résultats obtenus montrent que l'activité enzymatique de la SASPase est significativement augmentée en présence de concentration croissante de FLG2-Hs.

Des peptides de 8 à 20 acides aminés ont été synthétisés à partir de la séquence d'acides aminés s'étendant des positions 71 à 90 de la FLG2, SEQ ID NO: 9.

L'essai enzymatique précédent a été répété en remplaçant FLG2-Hs par chacun des peptides ainsi préparés.

Parmi les peptides testés, les peptides représentés par les séquences SEQ ID NO: 10, SEQ ID NO: 11, SEQ ID NO: 12, SEQ ID NO: 13, SEQ ID NO: 14 et SEQ ID NO: 15 se sont montrés dotés d'une capacité particulièrement importante pour augmenter l'activité catalytique de la SASPase.

### Exemple 3

*Criblage d'agents modulateurs de l'interaction entre un peptide issu de la FLG2 et la SASPase*

Un procédé selon l'invention peut mettre en oeuvre la technique de fluorescence homogène en temps résolu (ou HTRF).

Une séquence d'acides aminés issue de la SASPase (SEQ ID NO: 16) fusionnée à son extrémité C-terminale avec le marqueur d'affinité GST (Glutathion S-Transférase de *Schistosoma japonicum*) est préparé dans le vecteur pGEX-4-T3 (GenBank U13855) (SASPase$_{SEQ\ ID\ NO:\ 16}$-GST).

Une séquence d'acides aminés issue de FLG-2 (SEQ ID NO: 9) fusionnée à son extrémité N-terminale avec le marqueur d'affinité Thioredoxine-His-S.Tag est préparé dans comme suit (FLG-2$_{SEQ\ ID\ NO:\ 9}$-His-THX).

Les plasmides obtenus sont utilisés pour transformer des bactéries compétentes E. coli. Les bactéries transformées sont alors induites en présence d'IPTG, puis centrifugées et lysées par ultrason. Les peptides chimères sont purifiés par affinité pour le tag HIS ou GST sur une résine appropriée, puis dialysées contre un tampon. Les peptides chimères obtenus sont ensuite dosés par la méthode de Bradford.

Des anticorps reconnaissant, respectivement, les marqueurs d'affinité GST ou His-S.Tag, et porteurs, respectivement, d'un groupement donneur de fluorescence EuK (cryptate d'europium $\lambda_{ex}$ 337 nm/$\lambda_{em}$ 620 nm) ou d'un groupement receveur de fluorescence XL1665 ($\lambda_{ex}$ 570-630 nm/$\lambda_{em}$ 665 nm) sont utilisés pour suivre l'interaction peptide-SASPase.

De tels anticorps sont disponibles commercialement, par exemple auprès de la société CISCO sous les références 61GSTKLB (anticorps anti-GST K) ou 61HISKLB (anticorps anti-6HIS K).

L'interaction SASPase$_{SEQ\ ID\ NO:\ 16}$-GST/FLG-2$_{SEQ\ ID\ NO:\ 9}$-His-THX est observée par transfert d'énergie de fluorescence (FRET) entre les marqueurs fluorescents Euk et XL1665. Le donneur de fluorescence Euk est excité à $\lambda_{ex}$ 337 nm et l'émission de fluorescence de l'accepteur de fluorescence XL1665 est mesurée à $\lambda_{em}$ 665 nm.

L'émission de fluorescence Euk à $\lambda_{em}$ 620 nm, ayant lieu indépendamment de l'interaction entre les peptides, est utilisée pour normaliser le signal.

Le procédé de criblage est effectué en plaque 384 puits au moyen d'un automate Janus de Perkin Elmer dans un tampon phosphate PBS à pH 7,4.

Un tampon, ou une solution de dilution, dénué(e) de composé à cribler est utilisé(e) comme témoin.

La SASPase$_{SEQ\ ID\ NO:\ 16}$-GST et le composé testé sont pré-incubés, puis la FLG-2$_{SEQ\ ID\ NO:\ 9}$-His-THX est ajoutée au milieu d'incubation.

Les anticorps spécifiques des marqueurs d'affinité sont ensuite ajoutés au milieu d'incubation.

Le mélange est laissé incuber avant lecture de la fluorescence ($\lambda_{ex}$ 337 nm/$\lambda_{em}$ 665 nm, et normalisation à $\lambda_{em}$ 620 nm) au moyen d'un lecteur PHERASTAR (BMG).

Le calcul du résultat est effectué à l'aide de la formule suivante:

$$\text{Delta\_F} = 100 \times [\text{ratio}(100\%) - \text{ratio(background)}] / \text{ratio(background)}$$

dans laquelle « ratio(100%) » représente le rapport de fluorescence $\lambda_{em}665\lambda_{em}620$, des anticorps fluorescents en pré-

sence de SASPase$_{\text{SEQ ID NO: 8}}$-GST et FLG-2$_{\text{SEQ ID NO: 4}}$-His-THX, et « ratio(background) » représente le rapport de fluorescence $\lambda_{em}665/\lambda_{em}620$ des anticorps fluorescents en l'absence des peptides.

Delta_F est déterminé en présence « Delta_F Essai », ou en l'absence « Delta_F Témoin », des composés testés (contrôle en DMSO).

Le « ratio final » traduisant le pourcentage d'inhibition ou d'activation de l'interaction des peptides est ensuite calculé à l'aide de la formule suivante :

$$\text{Ratio final} = 100 \times (\text{Delta\_F Essai} - \text{Delta\_F Témoin})/(\text{Delta\_F témoin})$$

Une diminution de ce ratio final est indicative de la présence de composés aptes à prévenir ou déstabiliser l'interaction SASPase$_{\text{SEQ ID NO: 16}}$-GST/FLG-2$_{\text{SEQ ID NO: 9}}$-His-THX.

Une augmentation de ce ratio final est indicative de la présence de composés aptes à stabiliser ou favoriser cette interaction.

Le protocole de criblage de l'invention s'avère particulièrement avantageux pour identifier des composés actifs aptes à moduler positivement ou négativement l'interaction entre un peptide issu de la FLG2, et la SASPase ou un peptide issu de la SASPase.

Ces composés actifs criblés sont susceptibles d'être avantageusement utilisés à l'égard de désordres esthétiques ou pathologiques de la peau et/ou de ses annexes résultant d'un défaut de la prolifération et/ou de la différenciation des cellules de l'épiderme.

## Revendications

1.  Peptide isolé, représenté par une séquence d'acides aminés choisie parmi :

    - SEQ ID NO: 9,
    - un fragment de celle-ci choisi parmi les séquences SEQ ID NO : 10, SEQ ID NO : 11, SEQ ID NO : 12, SEQ ID NO : 13, SEQ ID NO : 14 et SEQ ID NO : 15, ou
    - un homologue de celle-ci présentant une identité de séquence d'au moins 85 % et apte à se lier par affinité à une séquence d'acides aminés issue d'une SASPase.

2.  Peptide selon la revendication 1, ledit peptide étant codé par une séquence d'acides nucléiques représentée par une séquence choisie parmi SEQ ID NO: 1, un fragment de celle-ci, ou un homologue de celle-ci présentant une identité de séquence d'au moins 85 %.

3.  Peptide chimère comprenant une première séquence d'acides aminés fusionnée à une seconde séquence d'acides aminés, dans lequel la première séquence d'acides aminés représente un peptide tel que défini selon la revendication 1 ou 2.

4.  Complexe isolé, formé par interaction d'une première séquence d'acides aminés avec une seconde séquence d'acides aminés issue d'une SASPase, ou d'un homologue de cette seconde séquence d'acides aminés présentant une identité de séquence d'au moins 85% avec la séquence SEQ ID NO : 16, dans lequel ladite première séquence d'acides aminés représente un peptide tel que défini selon une quelconque des revendications 1 à 3.

5.  Procédé de criblage *in vitro* ou *ex vivo* d'un composé étant un agoniste ou un antagoniste de l'interaction entre une première séquence d'acides aminés et une seconde séquence d'acides aminés issue d'une SASPase, ou d'un homologue de cette seconde séquence d'acides aminés présentant une identité de séquence d'au moins 85% avec la séquence SEQ ID NO : 16, dans lequel ladite première séquence d'acides aminés représente un peptide tel que défini selon une quelconque des revendications 1 à 3, ce composé étant également apte à réguler la prolifération et/ou la différenciation des cellules d'un épiderme, ledit procédé comprenant au moins les étapes consistant à :

    a) disposer d'un complexe tel que défini selon la revendication 4 dans des conditions propices à sa formation,
    b) associer, préalablement ou postérieurement à l'étape a), la première et/ou la seconde séquence(s) d'acides aminés à une entité,
    la première et/ou la seconde séquence(s) d'acides aminés formant avec ladite entité un ensemble apte à émettre un signal après exposition à une longueur d'onde excitatrice, et la réalisation des étapes a) et b) conduisant à l'obtention dudit complexe,
    c) soumettre ledit complexe à ladite longueur d'onde excitatrice pour obtenir un premier signal S1 caractéristique du complexe,
    d) déterminer quantitativement ou qualitativement le signal S1,
    e) mettre en contact le complexe avec un milieu présumé contenir un composé à cribler,
    f) soumettre le milieu obtenu à l'étape e) à la longueur d'onde excitatrice pour obtenir un second signal S2,
    g) déterminer quantitativement ou qualitativement le signal S2, et

h) comparer les premier et second signaux S1 et S2 pour tirer une conclusion relative à une éventuelle modification de la conformation tridimensionnelle du complexe en présence du composé criblé.

**6.** Procédé selon la revendication précédente, dans lequel ladite entité est choisie parmi un support convenant à la résonance plasmonique de surface, un marqueur fluorescent, un anticorps ou une combinaison d'anticorps primaire et secondaire, ledit anticorps ou ledit anticorps secondaire portant un marqueur fluorescent.

**7.** Procédé selon la revendication 5 ou 6, dans lequel la première et/ou la seconde séquence(s) d'acides aminés est/sont associée(s) à ladite entité par couplage chimique, par biologie moléculaire, ou par interaction(s) spécifique(s) de forte affinité.

**8.** Procédé de criblage selon l'une quelconque des revendications 5 à 7, dans lequel la première et la seconde séquences d'acides aminés sont associées, respectivement, à une première et une seconde entités, lesdites entités comportant, respectivement, un groupe fluorescent A et un groupe fluorescent B, lesdits groupes A et B définissant un couple accepteur-donneur d'énergie de fluorescence convenant à la mise en oeuvre d'un transfert d'énergie par résonance de fluorescence.

**9.** Procédé selon la revendication 8, **caractérisé en ce que** ledit signal S1 et/ou ledit signal S2 est un signal fluorescent obtenu par irradiation à une longueur d'onde excitatrice du donneur d'énergie de fluorescence.

**10.** Composition cosmétique comprenant dans un milieu physiologique acceptable une quantité efficace d'au moins un peptide tel que défini selon l'une quelconque des revendications 1 à 3 ou d'une séquence d'acides nucléiques codant pour un tel peptide.

**11.** Composition comprenant au moins une séquence d'acides nucléiques codant pour un peptide tel que défini selon l'une quelconque des revendications 1 à 3.

**Patentansprüche**

**1.** Isoliertes Peptid, dargestellt durch eine Aminosäuresequenz, ausgewählt aus:

- SEQ ID NO: 9,
- einem Fragment davon, ausgewählt aus den Sequenzen SEQ ID NO: 10, SEQ ID NO: 11, SEQ ID NO: 12, SEQ ID NO: 13, SEQ ID NO: 14 und SEQ ID NO: 15, oder
- einem Homolog davon, das eine Sequenzidentität von mindestens 85 % aufweist und zur Bindung an eine Aminosäuresequenz aus einer SASPase geeignet ist.

**2.** Peptid nach Anspruch 1, wobei das Peptid von einer Nukleinsäuresequenz kodiert wird, die durch eine Sequenz dargestellt wird, die ausgewählt ist aus SEQ ID NO: 1, einem Fragment davon oder einem Homolog davon, das eine Sequenzidentität von mindestens 85 % aufweist.

**3.** Chimäres Peptide, umfassend eine erste Aminosäuresequenz in Fusion mit einer zweiten Aminosäuresequenz, wobei die erste Aminosäuresequenz ein Peptid darstellt, wie nach Anspruch 1 oder 2 definiert.

**4.** Isolierter Komplex, der durch Wechselwirkung einer ersten Aminosäuresequenz mit einer zweiten Aminosäuresequenz aus einer SASPase gebildet wird, oder einem Homolog dieser zweiten Aminosäuresequenz, die eine Sequenzidentität von mindestens 85% mit der Sequenz SEQ ID NO: 16 aufweist, wobei die erste Aminosäuresequenz ein Peptid darstellt, wie nach einem der Ansprüche 1 bis 3 definiert.

**5.** In vitro oder ex vivo Screeningverfahren einer Verbindung, die ein Agonist oder Antagonist der Wechselwirkung zwischen einer ersten Aminosäuresequenz und einer zweiten Aminosäuresequenz aus einer SASPase, oder einem Homolog dieser zweiten Aminosäuresequenz, die eine Sequenzidentität von mindestens 85% mit der Sequenz SEQ ID NO: 16 aufweist, ist, wobei die erste Aminosäuresequenz ein Peptid, wie nach einem der Ansprüche 1 bis 3 definiert, darstellt, wobei diese Verbindung auch geeignet ist, die Proliferation und/oder Differenzierung von Zellen einer Epidermis zu steuern, wobei das Verfahren mindestens die folgenden Schritte umfasst:

a) Vorlegen eines Komplexes, wie nach Anspruch 4 definiert, unter Bedingungen, die seiner Bildung förderlich sind,

b) Kombinieren, vor oder nach Schritt a), der ersten und/oder der zweiten Aminosäuresequenz(en) mit einer Instanz,

wobei die erste(n) und/oder die zweite(n) Aminosäuresequenz(en) mit der Instanz eine Einheit bilden, die dazu ausgelegt ist, nach Exposition gegenüber einer Anregungswellenlänge ein Signal auszusenden, und wobei die Realisierung der Schritte a) und b) zum Erhalt des Komplexes führt,

c) Unterwerfen des Komplexes einer Anregungswellenlänge, um ein erstes, für den Komplex charakteristisches Signal S1 zu erhalten,

d) Bestimmen, quantitativ oder qualitativ, des Signals S1,

e) Inkontaktbringen des Komplexes mit einem Medium, das vermutlich eine zu srceenende Verbindung enthält,

f) Unterziehen des in Schritt e) erhaltenen Mediums der Anregungswellenlänge, um ein zweites Signal S2 zu erhalten,

g) Bestimmen, quantitativ oder qualitativ, des Signals S2, und

h) Vergleichen des ersten und zweiten Signals S1 und S2 zur Schlussfolgerung im Hinblick auf eine mögliche Modifikation der dreidimensionalen Konformation des Komplexes in Gegenwart der gescreenten Verbindung.

6. Verfahren nach dem vorangehenden Anspruch, wobei die Instanz ausgewählt ist aus einem Träger, der zur Oberflächenplasmonresonanz geeignet ist, einem Fluoreszenzmarker, einem Antikörper, wobei der Antikörper oder der sekundäre Antikörper einen Fluoreszenzmarker tragen.

7. Verfahren nach Anspruch 5 oder 6, wobei die erste(n) und/oder die zweite(n) Aminosäuresequenz(en) mit der Instanz durch chemische Bindung, molekularbiologisch oder durch spezifische Wechselwirkung(en) starker Affinität kombiniert wird (werden).

8. Screeningverfahren nach einem der Ansprüche 5 bis 7, wobei die erste und die zweite Aminosäuresequenz jeweils mit einer ersten und einem zweiten Instanz kombiniert werden, wobei die Instanzen jeweils eine fluoreszierende Gruppe A und eine fluoreszierende Gruppe B umfassen, wobei die Gruppen A und B eine fluoreszenzenergetische Akzeptor-Donor-Kopplung definieren, die zur Durchführung eines Resonanzenergietransfers geeignet ist.

9. Verfahren nach Anspruch 8, **dadurch gekennzeichnet, dass** das Signal S1 und/oder das Signal S2 ein Fluoreszenzsignal ist, das durch Bestrahlung bei einer Anregungswellenlänge des Fluoreszenzenergiedonors erhalten wird.

10. Kosmetische Zusammensetzung, umfassend in einem physiologisch verträglichen Medium einer wirksamen Menge von mindestens einem Peptid, wie nach einem der Ansprüche 1 bis 3 definiert, oder einer Nukleinsäuresequenz, die ein solches Peptide kodiert.

11. Zusammensetzung, umfassend mindestens eine Aminosäuresequenz, die ein Peptid, wie nach einem der Ansprüche 1 bis 3 definiert, kodiert.

## Claims

1. Isolated peptide, represented by an amino acid sequence selected from:

   - SEQ ID No.: 9,
   - a fragment thereof chosen from the sequences SEQ ID No.: 10, SEQ ID No.: 11, SEQ. ID No.; 12, SEQ ID No.; 13, SEQ ID No.: 14 and SEQ ID No.: 15, or
   - a homolog thereof having a sequence identity of at least 85% and capable of binding by affinity to an amino acid sequence derived from an SASPase.

2. Peptide according to claim 1, said peptide being encoded by a sequence of nucleic acids represented by a sequence selected from SEQ ID No.: 1, a fragment thereof or a homolog thereof having a sequence identity of at least 85%.

3. Chimeric peptide comprising a first amino acid sequence fused to a second amino acid sequence, wherein the first amino acid sequence represents a peptide as defined in claim 1 or 2.

4. Isolated complex, formed by interaction of a first amino acid sequence with a second amino acid sequence from an SASPase, or a homolog of this second amino acid sequence having a sequence identity of at least 85% with the sequence SEQ ID No.: 16, wherein said first amino acid sequence represents a peptide as defined in any one of the claims 1 to 3.

**5.** Method for *in vitro* or *ex vivo* screening of a compound being an agonist or antagonist of the interaction between a first amino acid sequence and a second amino acid sequence from an SASPase or a homolog of this second amino acid sequence having a sequence identity of at least 85% with the sequence SEQ ID No.: 16, wherein said first amino acid sequence represents a peptide as defined according to any one of the claims 1 to 3, wherein the compound is also capable of regulating the proliferation and/or differentiation of cells of an epidermis, wherein said method comprises at least the steps consisting in:

a) having a complex as defined according to claim 4 in conditions conducive to its formation,
b) associating, before or after step a), the first and/or second amino acid sequence(s) with an entity, wherein the first and/or second amino acid sequence(s) form with said entity an assembly capable of emitting a signal after exposure to an excitation wavelength, and implementing steps a) and b) leading to obtaining said complex,
c) subjecting said complex to said excitation wavelength to obtain a first signal S1 characteristic of the complex,
d) quantitatively or qualitatively determining the signal S1,
e) bringing the complex into contact with a medium presumed to contain a compound to be screened,
f) subjecting the medium obtained in step e) to the excitation wavelength to obtain a second signal S2,
g) quantitatively or qualitatively determining the signal S2, and
h) comparing the first and second signals S1 and S2 to draw a conclusion relating to a possible modification of the three-dimensional conformation of the complex in the presence of the screened compound.

**6.** Method according to the preceding claim, wherein said entity is chosen from a support which is suitable for surface plasmon resonance, a fluorescent label, an antibody or a combination of primary and secondary antibodies, said antibody or secondary antibody bearing a fluorescent label.

**7.** Method according to claim 5 or 6 wherein the first and/or second amino acid sequence(s) is/are associated with said entity by chemical coupling, by molecular biology, or by specific interaction(s) of strong affinity.

**8.** Screening method according to any one of the claims 5 to 7, wherein the first and second amino acid sequences are respectively associated with a first and a second entity, said entities respectively comprising a fluorescent group A and a fluorescent group B, wherein said groups A and B define a fluorescence energy acceptor-donor pairing suitable for the implementation of energy transfer by fluorescence resonance.

**9.** Method according to claim 8, **characterized in that** said signal S1 and/or said signal S2 is a fluorescent signal obtained by irradiation at an excitation wavelength for the fluorescence energy donor.

**10.** Cosmetic composition comprising in an acceptable physiological medium an effective amount of at least one peptide as defined in any one of the claims 1 to 3 or of a nucleic acid sequence encoding such a peptide.

**11.** Composition comprising at least one nucleic acid sequence encoding a peptide as defined in any one of the claims 1 to 3.

## LISTAGE DE SEQUENCES

### SEQ ID NO: 1 (*Homo sapiens* – ADNc - FLG2)

```
accgacctct tgagaagtgt tgtcaccgta attgatgttt tctacaaata caccaagcaa    60
gatggggagt gtggcacact gagcaagggt gaactaaagg aacttctgga gaaagagctt   120
catccagttc tgaagaaccc agatgatcca gacacagtgg atgtcatcat gcatatgctg   180
gatcgagatc atgacagaag attggacttt actgagtttc ttttgatgat attcaagctg   240
actatggcct gcaacaaggt cctcagcaaa gaatactgca aagct               285
```

### SEQ ID NO: 2 (*Homo sapiens* – ADNc - FLG2)

```
tttactgagt ttcttttgat gatattcaag ctgactatgg cctgcaacaa ggtcctcagc    60
```

### SEQ ID NO: 3 (*Homo sapiens* – ADNc - FLG2)

```
gagtttcttt tgatgatatt caagctgact atggcctgca acaag
```

### SEQ ID NO: 4 (*Homo sapiens* – ADNc - FLG2)

```
ctcatgatat tcaagctgac tatggcctgc aacaaggtcc tcagc               45
```

### SEQ ID NO: 5 (*Homo sapiens* – ADNc - FLG2)

```
ctcatgatat tcaagctgac tatggcctgc aacaaggtc                 39
```

### SEQ ID NO: 6 (*Homo sapiens* – ADNc - FLG2)

```
actgagtttc ttttgatgat attcaagctg actatggcct gcaacaag             48
```

### SEQ ID NO: 7 (*Homo sapiens* – ADNc - FLG2)

```
actgagtttc ttttgatgat attc                          24
```

### SEQ ID NO: 8 (*Homo sapiens* – ADNc - SASPase)

```
cggcagcatg ccttcgtccc ggaacctttt gatggggcca atgtcgtccc aaacctctgg    60
ctgcacagct ttgaagtcat caatgacctc aaccattggg accatatcac caagctaagg   120
ttcctgaaag agtccctcag aggagaggcc ctgggtgtct acaataggct cagtccccag   180
caccagggag actatgggac tgtgaaagag gccctcctga aggcctttgg g           231
```

**SEQ ID NO: 9** (*Homo sapiens* – protéine - FLG2)

```
        10         20         30         40         50         60
TDLLRSVVTV IDVFYKYTKQ DGECGYLSKG ELKELLEKEL HPVLKNPDDP DTVDVIMHML
```

```
        70         80         90
DRDHDRRLDF TEFLLMIFKL TMACNKVLSK EYCKA
```

**SEQ ID NO: 10** (*Homo sapiens* – protéine - FLG2)

```
        10         20
FTEFLLMIFK LTMACNKVLS
```

**SEQ ID NO: 11** (*Homo sapiens* – protéine - FLG2)

```
        10
EFLLMIFKLT MACNK
```

**SEQ ID NO: 12** (*Homo sapiens* – protéine - FLG2)

```
        10
LMIFKLTMAC NKVLS
```

**SEQ ID NO: 13** (*Homo sapiens* – protéine – FLG2)

```
        10
LMIFKLTMAC NKV
```

**SEQ ID NO: 14** (*Homo sapiens* – protéine – FLG2)

```
        10
TEFLLMIFKL TMACNK
```

**SEQ ID NO: 15** (*Homo sapiens* – protéine – FLG2)

```
TEFLLMIF
```

**SEQ ID NO: 16** (*Homo sapiens* – protéine - SASPase)

```
        10         20         30         40         50         60
FQHAFVPEPF DGANVVPNLW LHSFEVINDL NHWDHITKLR FLKESLRGEA LGVYNRLSPQ
```

```
        70
DQGDYGTVKE ALLKAFG
```

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2842209
- FR 1060177
- US 61457090 B

**Littérature non-brevet citée dans la description**

- **TOULZA et al.** *Genome Biology,* 2007, vol. 8 (6
- **KYTE ; DOOLITTLE.** *J. Mol. Biol.,* 1982, vol. 157, 105
- **SAMBROOK et al.** Molecular Cloning : A laboratory Manual. Cold Spring Harbor, 2001
- Antibodies : A Laboratory Manual. Cold Spring Harbor Laboratory Press, 1990
- **BERNARD et al.** *J. Invest. Dermatol.,* 2005, vol. 125, 278-287